# EUROPEAN PATENT SPECIFICATION

(11) **EP 1 405 857 B1**
(45) Date of publication and mention of the grant of the patent: **15.05.2013**
(21) Application number: 02738865.1
(22) Date of filing: 28.06.2002
(51) Int. Cl.: C07H 9/06, C07H 1/00

(54) **Process for the production of 2-methyl-(3,4,6-tri-O-acetyl-1,2-dideoxy-alpha-glucopyrano)-[2,1-d]-2-oxazoline**
Verfahren zur Herstellung von 2-Methyl-(3,4,6-tri-O-acetyl-1,2-didesoxy-alpha-glucopyrano)-[2,1-d]-2-oxazolin
Procédé de production de 2-méthyl-(3,4,6-tri-O-acetyl-1,2-didéoxy-alpha-glucopyrano)-[2,1-d]-2-oxazoline

(30) Priority: 02.07.2001 JP 2001201316
(43) Date of publication of application: 07.04.2004
(73) Proprietor: SEIKAGAKU CORPORATION, Chiyoda-ku, Tokyo 100-0005 (JP)
(72) Inventor: SHODA, Shin-ichiro, Aoba-ku, Sendai-shi, Miyagi 980-0021 (JP); FUJITA, Masaya, Koganei-shi Tokyo 184-0011 (JP); SUENAGA, Masako, Mobara-shi, Chiba 297-0017 (JP); SAITO, Kenji, Sendai-shi, Miyagi 984-0838 (JP)
(74) Representative: Elsy, David
(86) International application number: PCT/JP2002/006575
(87) International publication number: WO 2003/004508

(56) References cited:
- JP-A- 8 245 678
- JP-A- 9 003 088
- US-A- 5 382 665
- KOBAYASHI, SHIRO ET AL: "A novel method for the synthesis of chitobiose via enzymatic glycosylation using a sugar oxazoline as glycosyl donor" TETRAHEDRON LETTERS, vol. 38, no. 12, 1997, pages 2111-2112, XP002443800
- WONG, WAI C ET AL.: "A convenient synthesis of 2-amino-2-oxazolines and their pharmacological evaluation at cloned human alpha adrenergic receptors" BIOORGANIC & MEDICINAL CHEMISTRY LETTERS, vol. 4, no. 19, 1994, pages 2317-2322, XP002443801
- MITCHELL M.A. ET AL.: 'Synthesis of delta2-1,3-oxazolines and delta2-1,3-oxazines using potassium fluoride on alumina' SYNTHESIS 1994, pages 675 - 677, XP002956187
- SHIN-ICHIRO SHODA ET AL.: 'A facile method for synthesis of 1,2-oxazoline derivative of N-acetylglucosamine promoted by potassium fluoride' CHEMISTRY LETTERS no. 2, 05 February 2002, pages 150 - 151, XP002956188

## Description

### Technical Field

The present invention relates to a process for the production of a sugar oxazoline derivative.

### Background Art

In recent years, much attention is given to sugar oxazoline derivatives as substrates for glycosylation using sugar-chain related enzymes, and methods for obtaining sugar oxazoline derivatives are investigated. Conventionally, the process for the production of bicyclic sugar oxazoline derivatives, in which a reaction is carried out by adding sodium bicarbonate as an acid trapping agent to an acetonitrile solution of N-acetyl-3,4,6-tri-O-acetyl-a-glucosaminyl chloride using tetraethylammonium chloride as a nucleophilic reagent, has been used (JP 09-3088 A) . In this process, however, it is difficult to remove an excessive amount of the reaction agent remaining in the solution after completion of the reaction. Thus, complicated purification procedures are required for obtaining sugar oxazoline derivatives of high purity.

KOBAYASHI, SHIRO ET AL, "A novel method for the synthesis of chitobiose via enzymatic glycosylation using a sugar oxazoline as glycosyl donor" TETRAHEDRON LETTERS, vol. 38, no. 12, 1997, pages 2111-2112, describes the formation of a sugar oxazoline ring from peracetyl glycosyl chloride as a starting material by the action of base in the presence of ammonium chloride.

WONG, WAI C ET AL, "A convenient synthesis of 2-amino-2-oxazolines and their pharmacological evaluation at cloned human alpha adrenergic receptors" BIOORGANIC & MEDICINAL CHEMISTRY LETTERS, vol. 4, no. 19, 1994, pages 2317-2322, describes the formation of oxazoline rings by potassium fluoride through the use of n-(2-chloroethyl) amide.

### Disclosure of the Invention

In recent years, the actions of oligosaccharides, glycosaminoglycans, and so on, which are *in vivo* materials, are attracting attention in the medical field. Therefore, there is a need of an industrializable, cost effective, and simpler process for the production of sugar oxazoline derivatives to serve as substrates for enzymatic synthesis of the oligosaccharides and glycosaminoglycans.

The inventors of the present invention have made extensive studies with a view to overcome the above-mentioned problems. As a result, they have found out that if a metal fluoride is used as a reaction agent, the fluoride exerts both nucleophilic and acid-trapping actions to expedite the synthesis of sugar oxazoline derivatives, and that the fluoride can be removed easily, thus completing the present invention.

According to the present invention, there is provided a process for production of a sugar oxazoline derivative represented by the following general formula (2), comprising the step of reacting an acylamino sugar represented by the following general formula (1) with a metal fluoride; 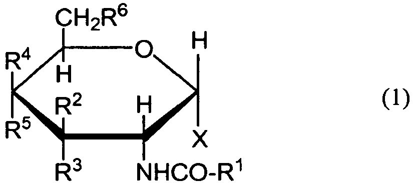 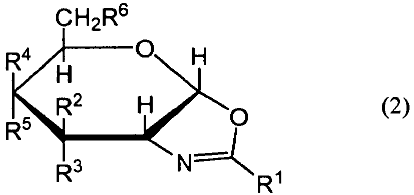 wherein the acylamino sugar represented by the general formula (1) is N-acetyl-3,4,6-tri-O-acetyl-α-glucosaminyl chloride and the sugar oxazoline derivative represented by the general formula (2) is 2-methyl-(3,4,6-tri-O-acetyl-1,2-dideoxy-α-glucopyrano-[2,1-d]-2-oxazoline.

Further, the present invention is a method for production of a sugar oxazoline derivative represented by the following general formula (3), comprising the steps of:
producing the sugar oxazoline derivative represented by the general formula (2) by the method above and
removing at least a part of a protective group of the resulting sugar oxazoline derivative; 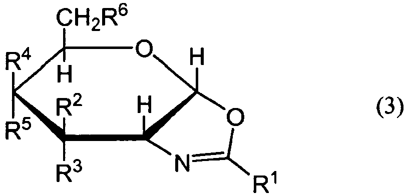
wherein R¹ is CH₃,
R², R⁵, and R⁶ are each independently selected from OH or O-acetyl; and R³ and R⁴ are H.

In the above-described production processes, it is preferable the the metal fluoride be an alkali metal fluoride.

### Best Mode for carrying out the Invention

The process for production of the sugar oxazoline derivative represented by the general formula (2) of the present invention comprises the step of reacting an acylamino sugar represented by the general formula (1) with a metal fluoride.

The metal fluoride to be used in the production process of the present invention is not particularly limited, but is preferably an alkaline metal fluoride, and more preferably sodium fluoride, potassium fluoride, rubidium fluoride, cesium fluoride, or the like. At the time of the production on an industrial scale, sodium fluoride and potassium fluoride are particularly preferable in consideration of handling, price, and so on as a reagent.

The metal fluoride may be held on an inorganic solid carrier likewise with the conventional process. Examples of the inorganic solid carrier include alumina, silica gel, magnesium oxide, molecular sieves (e.g., Linde 4A (trade name)), clay (e.g., montmorillonite), and diatomaceous earth (e.g., Celite (trade name). Among them, alumina is particularly preferable.

The conditions for reaction between the acylamino sugar represented by the general formula (1) and the metal fluoride are not specifically limited provided that a bicyclic sugar oxazoline derivative can be generated by the reaction between the acylamino sugar and the metal fluoride, which are used in the present invention, and thus the conditions can be suitably set by a person skilled in the art.

A solvent to be used in the reaction is not particularly limited provided that the solvent do not react with the acylamino sugar and the metal fluoride to be used and is capable of dissolving the acylamino sugar therein. As the solvent, acetonitrile (CH₃CN), dimethyl sulfoxide (DMSO), dimethylformamide (DMF), tetrahydrofuran, xylene, and the like are preferable, and among them, CH₃CN, DMSO, and DMF are more preferable. In particular, CH₃CN is preferable.

The amount of metal fluoride to be used and the reaction conditions such as a reaction temperature and a reaction time can be suitably set depending on the amount of acylamino sugar to be used and so on. A molar ratio between the acylamino sugar and the metal fluoride is preferably 1 : 2 to 1 : 30, more preferably 1 : 10. The reaction temperature is preferably from room temperature to a boiling point of the solvent, more preferably from 30 to 60°C. Furthermore, in the case of carrying out the reaction at the boiling point of the solvent to be used, a reflux condenser or the like may be used. The reaction time is preferably 0.5 hours to three days.

At the time of setting the reaction conditions, it is preferable to confirm the completion of the reaction. A concrete example of a method for confirming the completion of the reaction is thin-layer chromatography.

A reaction between the acylamino sugar represented by the general formula (1) and the metal fluoride is preferably performed under an atmosphere of argon, nitrogen, or the like to avoid the reaction with water.

As a method for purifying a sugar oxazoline derivative as a target material from a reaction-completed solution, any of the purification processes conventionally performed may be suitably selected and used. For example, after removal of an insoluble matter from the reaction-completed solution, a water-soluble material being dissolved in the reaction-completed solution was removed by a liquid-separating operation, followed by purifying with silica-gel chromatography, recrystallization, or the like.

A method for removing an insoluble matter from the reaction-completed solution has only to divide an insoluble matter (solid) and a solution (liquid) and thus may be a method including a filtration process using a glass filter, celite, or the like, which is generally used in the art.

The process for production of the sugar oxazoline derivative represented by the general formula (3) is characterized in that the sugar oxazoline derivative represented by the general formula (2) is obtained by the above method and at least a part of the protective group of the obtained sugar oxazoline derivative is removed. In this process, the sugar oxazoline derivative represented by the general formula (3) can be obtained, in which at least a part of the protective group of the sugar oxazoline derivative represented by the general formula (2) is removed. The protective group can be removed by the conventional method.

The sugar oxazoline derivative obtained by the production process of the present invention may be used as a substrate for the synthesis of a polymer using the ring-opening polymerization of an oxazoline ring.

### Examples

Hereinafter, the present invention will be described more concretely on the basis of examples.

### Example 1: Synthesis of bicyclic oxazoline using metal fluoride

Under each of the reaction conditions described in Table 1 below, the synthesis was carried out by the following procedures.

Under an argon atmosphere, 75 ml of an acetonitrile solution of 1.8 g (5 mmol) N-acetyl-3,4,6-tri-O-acetyl-α-glucosaminyl chloride or 75 ml of a dimethylformamide solution of 1.8 g (5 mmol) N-acetyl-3,4,6-tri-O-acetyl-α-glucosaminyl chloride was added to potassium fluoride or cesium fluoride to carry out a reaction under reflux. The completion of the reaction was confirmed by thin-layer chromatography, followed by filtration with a glass filter G4. Then, the resultant filtrate was condensed with an evaporator and was diluted with an excess amount of chloroform. Until the pH of the solution became neutral, a liquid-separating operation was performed with a sodium bicarbonate saturated solution and subsequently with cold water. The collected organic layer was dried on anhydrous sodium sulfate. Then, the sodium sulfate was removed by filtration and the filtrate was condensed with an evaporator. The obtained residue was purified by silica-gel flash chromatography (gel: Silika gel 60 manufactured by Merck Co., Ltd., 0.040 to 0.063 mm in particle size, developing solvent: hexane/ethyl acetate = 2/3 (volume ratio)). The solution after the development was further condensed with the evaporator and was dried under reduced pressure, resulting in 2-methyl (3,4,6-tri-O-acetyl-1,2-dideoxy-a-glucopyrano) [2,1-d]-2-oxazoline in the form of yellow syrup. The yield thereof is shown in Table 1.

**Table 1**

| Metal fluoride | Equivalent^{*1} | Solvent | Reaction temperature (°C) | Reaction time (hours) | Yield (%) |
|---|---|---|---|---|---|
| KF | 1 | CH₃CN | 60 | 24 | 71.4 |
| | 5 | CH₃CN | 60 | 78 | 81.3 |
| | 10 | CH₃CN | 60 | 88 | 78.7 |
| | 10 | DMF | 60 | 24 | 66.2 |
| CsF | 1 | CH₃CN | Room temp. | 24 | 9.5 |
| | 5 | CH₃CN | Room temp. | 24 | 10.0 |
| | 10 | CH₃CN | 60 | 1 | 34.7 |
| | 10 | DMF | Room temp. | 24 | 18.5 |

| | | | | | |
|---|---|---|---|---|---|
| *1: Equivalent to N-acetyl-3,4,6-tri-O-acetyl-α-glucosaminyl chloride (10 equivalents of potassium fluoride is 2.9 g and 10 equivalents of cesium fluoride is 7.6 g) | | | | | |

The NMR data of the resulting product (2-methyl (3,4,6-tri-O-acetyl-1,2-dideoxy-α-glucopyrano) [2,1-d]-2-oxazoline) was as follows:
¹H NMR (250 MHz, CDCl₃) : 5.95 (1H, d, J_{1,2} = 7.34 Hz, anomeric proton), 2.0-2.1 (12H, m, methyl proton of acetate and methyl proton of oxazoline); and
¹³C NMR (62.9 MHz, CDCl₃) : 99.4 (C-1), 20.7-20.9 (3C, methyl C of acetate), 13.9 (methyl C of oxazoline), 169.2-170.6 (3C, carbonyl C of acetyl), 166.7 (C of oxazoline ring: O-C=N).

### Industrial Applicability

According to the present invention, there is provided a mass-productive, cost effective, and simpler process for the production of sugar oxazoline derivatives.

## Claims

1. A method for production of a sugar oxazoline derivative represented by the following general formula (2), comprising the step of:
reacting an acylamino sugar represented by the following general formula (1) with a metal fluoride; 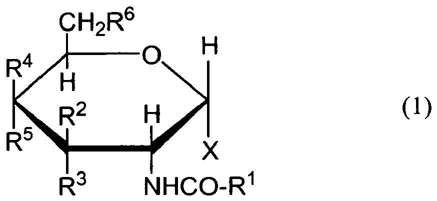 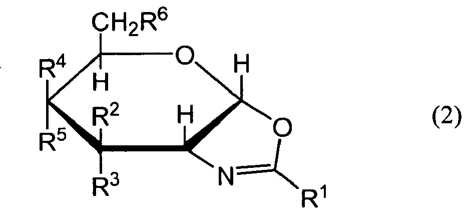 wherein the acylamino sugar represented by the general formula (1) is N-acetyl-3,4,6-tri-O-acetyl-α-glucosaminyl chloride and the sugar oxazoline derivative represented by the general formula (2) is 2-methyl-(3,4,6-tri-O-acetyl-1,2-dideoxy-α-glucopyrano-[2,1-d]-2-oxazoline.

2. The method for production according to Claim 1,
wherein the metal fluoride is an alkali metal fluoride.

3. A method for production of a sugar oxazoline derivative represented by the following general formula (3), comprising the steps of:
producing the sugar oxazoline derivative represented by the general formula (2) by the method according to claim 1 or claim 2; and
removing at least a part of a protective group of the resulting sugar oxazoline derivative; 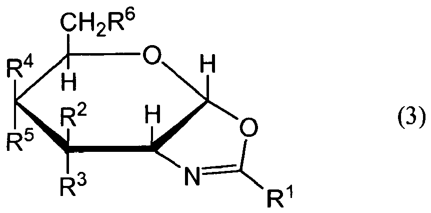 wherein R¹ is CH₃,
R², R⁵, and R⁶ are each independently selected from OH or O-acetyl; and R³ and R⁴ are H.

## Patentansprüche

1. Ein Verfahren zur Herstellung eines Zucker-Oxazolin-Derivats, das durch die folgende allgemeine Formel (2) dargestellt ist, umfassend die Schritte:
Reagieren eines Acylamino-Zuckers, der durch die folgende allgemeine Formel (1) dargestellt ist, mit einem Metallfluorid, 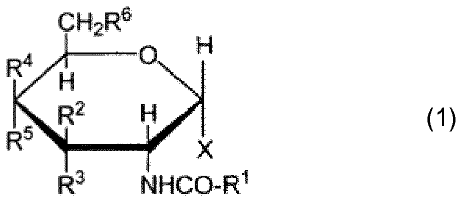 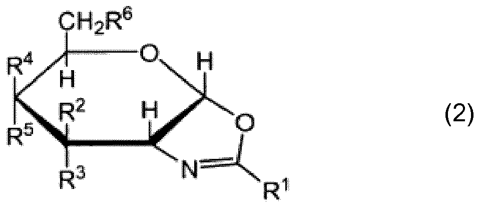 wobei der Acylamino-Zucker, der durch die allgemeine Formel (1) dargestellt ist, N-Acetyl-3,4,6-Tri-O-Acetyl-α-Glukosaminyl-Chlorid ist und das Zucker-Oxazolin-Derivat, das durch die allgemeine Formel (2) dargestellt ist, 2-Methyl-(3,4,6-Tri-O-Acetyl-1,2-Dideoxy-α-Glukopyrano-[2,1-d]-2-Oxazolin ist.

2. Das Herstellungsverfahren nach Anspruch 1, wobei das Metallfluorid ein Alkalimetallfluorid ist.

3. Ein Verfahren zur Herstellung eines Zucker-Oxazolin-Derivats, das durch die folgende allgemeine Formel (3) dargestellt ist, umfassend die Schritte:
Herstellen des Zucker-Oxazolin-Derivats, das durch die allgemeine Formel (2) dargestellt ist, durch das Verfahren nach Anspruch 1 oder Anspruch 2 und
Entfernen mindestens eines Teils einer Schutzgruppe des resultierenden Zucker-Oxazolin-Derivats, 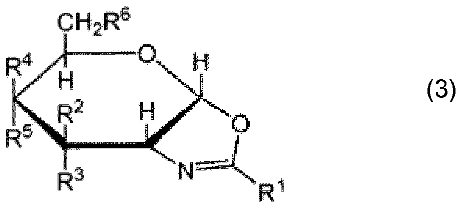 wobei R¹ CH₃ ist,
R², R⁵ und R⁶ jeweils unabhängig voneinander aus OH oder O-Acetyl ausgewählt sind und R³ und R⁴ H sind.

## Revendications

1. Procédé de production d'un dérivé de sucre oxazoline répondant à la formule (2) ci-dessous comprenant les étapes consistant à :
faire réagir un acylamino sucre représenté par la formule générale (1) ci-dessous avec un fluorure métallique, 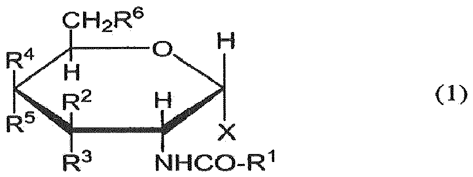 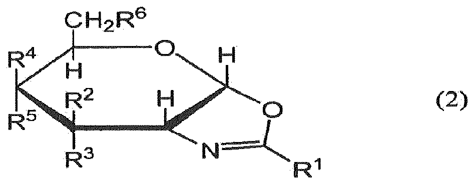
formules dans lesquelles l'acylamino sucre représenté par la formule (1) est le chlorure de N-acétyl-3,4,6-tri-O-acétyl-a-glycosaminyl et le dérivé de sucre oxazoline représenté par la formule générale (2) est la 2-méthyl-(3,4, 6-tri-O-acétyl-1,2-dideoxy-a-glucopyrano-[2,1-d]-2-oxazoline.

2. Procédé de production conforme à la revendication 1, selon lequel le fluorure métallique est un fluorure de métal alcalin.

3. Procédé de production d'un dérivé de sucre oxazoline représenté par la formule générale (3) ci-dessous comprenant les étapes consistant à :
produire le dérivé de sucre oxazoline représenté par la formule générale (2) par la mise en oeuvre du procédé conforme à la revendication 1 ou 2, et
éliminer au moins une partie d'un groupe protecteur du dérivé de sucre oxazoline résultant, 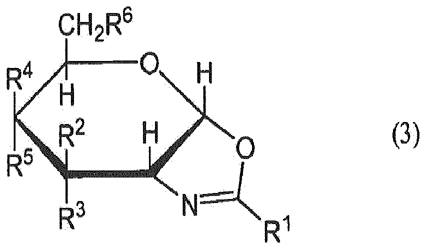
formule dans laquelle R₁ représente CH₃,
R₂, R₅ et R₆ sont indépendamment choisis parmi les groupes OH et O-acétyle, et R₃ et R₄ représente H.
